# EUROPEAN PATENT APPLICATION

(11) **EP 4 534 012 A1**
(43) Date of publication of application: **09.04.2025**
(21) Application number: 23827007.8
(22) Date of filing: 09.06.2023
(51) Int. Cl.: A61B 5/22, A61B 5/11

(54) **MANUAL MUSCLE STRENGTH TESTING DEVICE**

(30) Priority: 22.06.2022 JP 2022100641
(71) Applicant: Yamaguchi University, Yamaguchi 753-8511 (JP)
(72) Inventor: TAKESHITA, Yukio, Ube-shi, Yamaguchi 755-8505 (JP); JIANG, Zhongwei, Ube-shi, Yamaguchi 755-8611 (JP); KAWAMURA, Ryutaro, Ube-shi, Yamaguchi 755-8611 (JP); TAKATA, Hiroshi, Ube-shi, Yamaguchi 755-8505 (JP)
(74) Representative: Vossius & Partner Patentanwälte Rechtsanwälte mbB
(86) International application number: PCT/JP2023/021448
(87) International publication number: WO 2023/248820

(57) **Abstract**

The purpose of the present invention is:
to providing a manual muscle strength testing device with which it is possible, by using an inertial sensor and a load exercise measurement device of simple configuration, to capture very small muscle contractions of the fingers while suppressing the compensatory effects of surrounding muscles; and to make it possible to evaluate the state or the degree of recovery of the median, ulnar, and radial nerves by measuring only a load exercise of the index finger.

A manual muscle strength testing device according to the present invention has, inter alia:
a finger insertion portion 30, comprising an other finger fixation portion 28 having a space that is shaped so as to prevent movement, in any direction, of a middle, a ring, or little finger inserted thereinto, and an index finger insertion portion 29 having a space that is shaped such that an inserted index finger is able to move towards the ventral side or the thumb side, or the nail side or the thumb side; a ring 15 that can be fitted onto the fingertip of the index finger; a pressure sensor 32 capable of measuring the magnitude of a load applied on the fingertip and a 3-axis acceleration sensor 11 capable of measuring the movement of the fingertip, fixed to the ring 15; and a load elastic body 31 that applies an adjustable load to the fingertip on which the ring 15 is worn.

## Description

### Field

This invention relates to a manual muscle strength testing device that can evaluate a state and a recovery level of median, ulnar, and radial nerves.

### Background

In recent years, inventions of remedies and developments of regenerative medicine for various central and peripheral nerve diseases have rapidly changed the era in which "nerve diseases are incurable" to the era in which "nerve diseases are curable by selecting therapies suitable for the pathology". In contrast, damaged nerve fibers recover extremely slowly, and it is thus important to evaluate very small recovery of nerves through therapies.

Current evaluation methods of nerves actually used in clinical settings are broadly divided into two methods.

One evaluation method is based on testing with a grip strength meter (hereinafter referred to as "grip strength testing"), and the other evaluation method is based on manual muscle strength testing (hereinafter referred to as "MMT")**.**

The former is a method that evaluates a state and a recovery level of nerves in a patient by visualizing variation of grip strength values based on daily measurement of the grip strength of the patient and recording the measurement results.

The latter evaluation method using the MMT is a method that measures the degree of a nerve damage by a physician touching the site of a nerve disease of a patient with a hand and grasping the state of exercise while a load is applied to a skeleton of the patient. The evaluation of the degree of the nerve damage is divided into the following six levels (MMT 0 to 5) based on the subjectivity of the physician. 0 (Zero): a state where a force is not exerted and muscles do not move at all even if the patient intends to move the muscles.
1 (Trace): a state where muscle contraction occurs when the patient strains, but joints cannot be moved.
2 (Poor): a state where a force sufficient to move a joint is exerted (when the moving direction of the joint is approximately horizontal to the ground) if the effect of the gravity is excluded.
3 (fair): a state where a force sufficient to move a joint is exerted (even if the joint moves in an approximately vertical direction to the ground) against the gravity.
4 (good): a state where a joint can be moved firmly even if some resistance is applied thereto.
5 (Normal): a state where muscle strength that can move a joint completely is exerted even if significant resistance is applied.

Unfortunately, the evaluation method of nerves based on the grip strength testing and the evaluation method of nerves based on MMT have their own problems and a common problem.

The grip strength testing has the following problems: "grip strength meters are designated for healthy subjects and cannot measure slight grip strength of a patient"; and "measured values of grip strength meters represent the sum of strength of a median nerve-innervated muscle and an ulnar nerve-innervated muscle, which thus cannot evaluate muscle strength for each of the nerves (median, ulnar, and radial nerves)". For example, when the median nerve is normal and only the ulnar nerve is damaged, grip strength meters mainly measure muscle strength innervated by normal median nerves, and thus cannot evaluate the state and the recovery of the ulnar nerve-innervated muscle.

Next, the MMT has the following problems: "measurement results are not quantitative and objective because the measurement is based on subjective classification of degree of a nerve damage by an individual and subjectively depends on the skill of a physician"; and "damaged nerve fibers recover very slowly, so that a slight recovery level of nerves thus cannot be evaluated in six levels". Therefore, there is an apparent problem in which, even if the nerve of a patient is in a recovery process, symptomatic improvement cannot be evaluated correctly or reported to the patient with objective indices, and the therapy is discontinued.

A problem common to both of the tests is that "the grip strength testing and the MMT each measure maximum muscle strength and are unsuitable for clinical settings focusing on evaluation of the state and the recovery of each nerve".

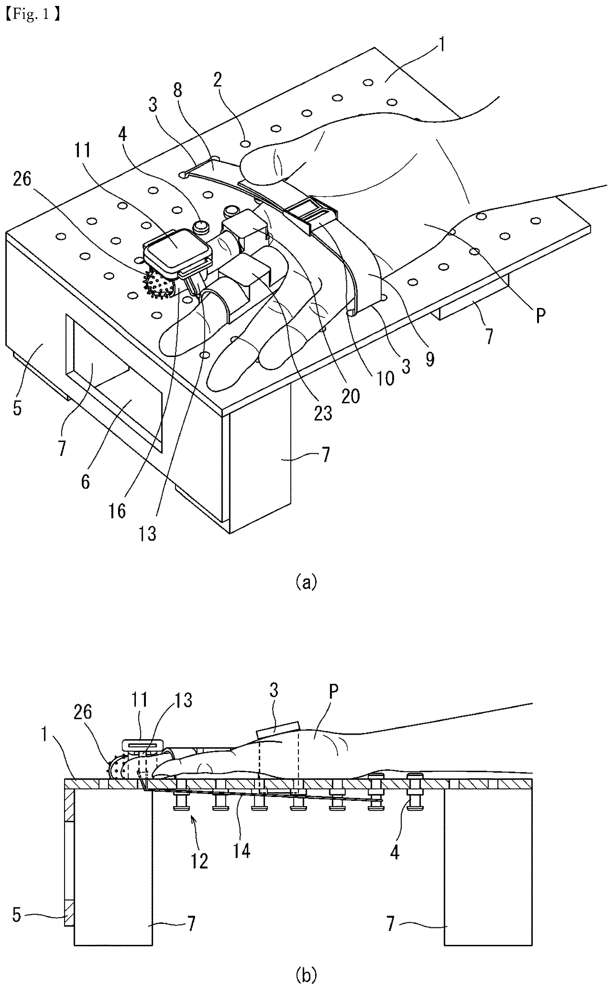

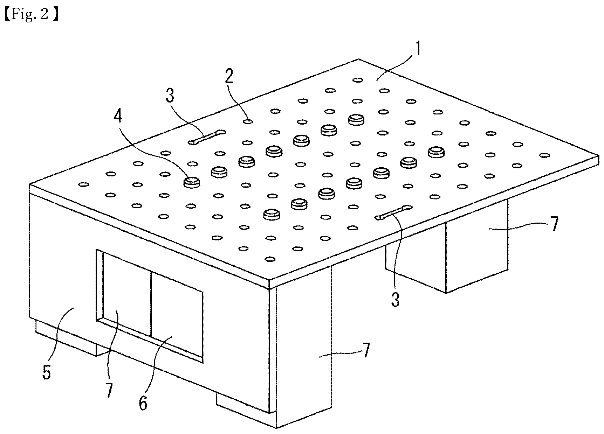

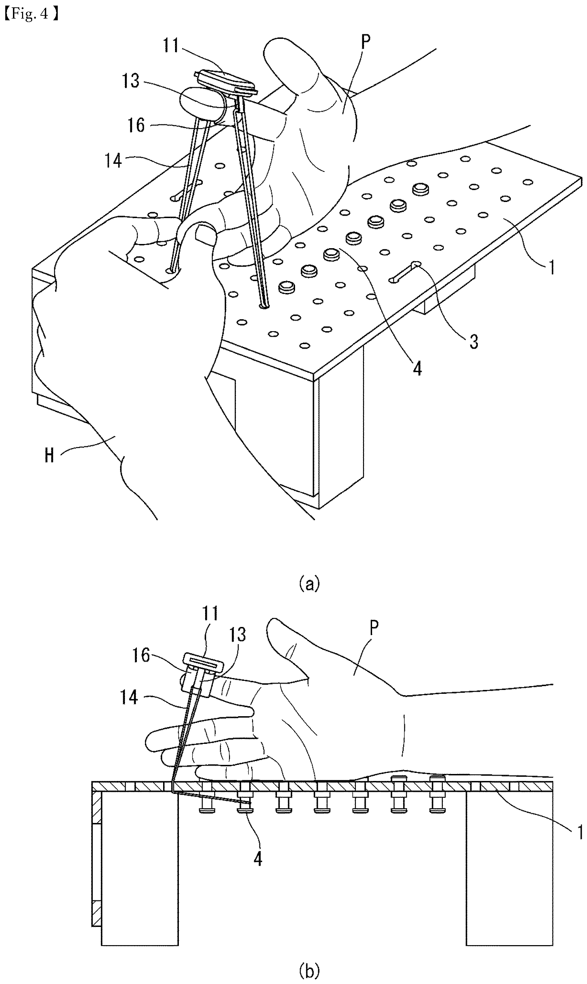

Patent Literature 1 (JP 2014-8324 A) describes an evaluation system of a thumb and other fingers. The system is a measuring device (10) for evaluating the maximum muscle strength of individual fingers, including a fixation base (12) on which an arm (2) is fixed to suppress the effects of arm strength, a sensor (11) into which a finger is inserted to measure the muscle strength, and other components (in particular, see paragraph 0009, FIGS. 1 and 4).
Patent Literature 2 (JP 2016-83004 A) describes a health monitoring system including a movement detector (1) that detects the movement of fingers of a user and transmits measurement data, and a management device (2) that analyzes the movement of the fingers based on the measurement data and monitors the health status of the user (in particular, see paragraph 0016 and FIG. 1). The movement detector (1) includes a plurality of movement detecting sensors (3) and a communication device (4) that is connected to the movement detecting sensors (3) and communicates with the management device (2). The movement detecting sensors (3) each include a base (5), a pair of arms (6), an acceleration sensor (7) that detects acceleration of a finger, and a contact pressure sensor (8) that detects pressure when the finger touches an object. The movement detecting sensors (3) are worn on at least three fingers (thumb, little finger, index finger, or middle finger), the detection of the movement of the thumb and the index or middle finger is used for determination of median nerve damages, the detection of the movement of the little finger is used for determination of ulnar nerve damages, and the detection of the movement of the thumb and the index finger is used for determination of radial nerve damages (in particular, see paragraphs 0017 and 0027, FIGS. 2 to 4).

### Citation List

### Patent Literature

Patent Literature 1: JP 2014-008324 A (JP 5958962 B2)
Patent Literature 2: JP 2016-083004 A (JP 6546733 B2)

### Summary

### Technical Problem

The system for evaluating a thumb and other fingers described in Patent Literature 1 contributes to the evaluation of muscle strength and exercise coordination of the thumb and the other fingers and rehabilitation of the fingers, as described in paragraph 0012. Unfortunately, as with the problem of grip strength testing, the system cannot evaluate muscle strength for each of the nerves (median, ulnar, and radial nerves), and is for measuring maximum muscle strength as with grip strength testing and MMT. The system is thus unsuitable for clinical settings focusing on the evaluation of the state and the recovery of each nerve.

Although the health monitoring system described in Patent Literature 2 is used for determination of damages of median, ulnar, and radial nerves as described in paragraph 0027, the movement detector (1) has the plurality of movement detecting sensors (3), the management device (2), and the communication device (4), and the movement detecting sensors (3) each have the base (5), the pair of arms (6), the acceleration sensor (7), and the contact pressure sensor (8), and need to be worn on at least three fingers (thumb, little finger, index finger, or middle finger). Thus the health monitoring system described in Patent Literature 2 has the following problems: the system structure is complex and expensive; and the monitoring is performed while each finger can be freely moved, thereby being susceptible to the effect of strength of the arm and other fingers.

In order to solve the problems of grip strength testing and MMT actually used in clinical settings and the problems of Patent Literatures 1 and 2, the first object of this invention is to provide a manual muscle strength testing device that can capture very small muscle contractions of a finger with a simple load exercise measurement device and a small, lightweight inertial sensor while suppressing compensatory effects of surrounding muscles. The second object of this invention is to enable evaluation of the state and the recovery level of the median, ulnar, and radial nerves by measuring only a load exercise of the index finger with the manual muscle strength testing device.

### Solution to Problem

A manual muscle strength testing device of an invention according to claim 1 comprising:
an other-finger fixation portion capable of fixing a middle finger, a ring finger, and a little finger of a subject;
an inertial sensor wearable on a fingertip of an index finger of the subject, being capable of measuring movement of the fingertip and recording or transmitting movement measurement data; and
a load applying portion that applies a load to the fingertip wearing the inertial sensor.

An invention according to claim 2 is the manual muscle strength testing device according to claim 1, wherein the load applying portion is either an elastic body that is fixed to a ventral side of the fingertip and contracts with a force in a direction the fingertip moves to the ventral side, an elastic body that is fixed to a nail side of the fingertip and extends with a force in a direction the fingertip moves away from the nail side, an elastic body that is fixed to a thumb side of the fingertip and contracts with a force in a direction the fingertip moves to the thumb side, an elastic body that is fixed to an other-finger fixation portion side of the fingertip and extends with a force in a direction the fingertip moves away from the other-finger fixation portion, an elastic body that is fixed to the nail side of the fingertip and contracts with a force in a direction the fingertip moves to the nail side, or an elastic body that is fixed to the ventral side of the fingertip and extends with a force in a direction the fingertip moves away from the ventral side.

An invention according to claim 3 is the manual muscle strength testing device according to claim 1 or 2, comprising a pressure sensor wearable on the fingertip, being capable of measuring magnitude of a load applied to either the ventral side, the thumb side, or the nail side of the fingertip and recording or transmitting load measurement data, wherein
the inertial sensor and the pressure sensor are fixed to a ring wearable on the fingertip,
the pressure sensor is fixed to all of or any one of the ventral side, the thumb side, and the nail side of the fingertip at an outside circular portion of the ring, and
the load applying portion applies a load to the pressure sensor fixed to either the ventral side, the thumb side, or the nail side of the fingertip.

### Advantageous Effects of Invention

The manual muscle strength testing device of the invention according to claim 1 can provide a manual muscle strength testing device that is able to capture very small muscle contractions of a finger with a simple load exercise measurement device and a small, lightweight inertial sensor while suppressing compensatory effects of surrounding muscles.

In the manual muscle strength testing device of the invention according to claim 2, in addition to the effect of the invention according to claim 1, the load applying portion is either the elastic body that is fixed to the ventral side of the fingertip and contracts with the force in the direction the fingertip moves to the ventral side, the elastic body that is fixed to the nail side of the fingertip and extends with the force in the direction the fingertip moves away from the nail side, the elastic body that is fixed to the thumb side of the fingertip and contracts with the force in the direction the fingertip moves to the thumb side, the elastic body that is fixed to the other-finger fixation portion side of the fingertip and extends with the force in the direction the fingertip moves away from the other-finger fixation portion, the elastic body that is fixed to the nail side of the fingertip and contracts with the force in the direction the fingertip moves to the nail side, or the elastic body that is fixed to the ventral side of the fingertip and extends with the force in the direction the fingertip moves away from the ventral side. Therefore, the load applying portion is able to apply an approximately constant load to the fingertip when the fingertip moves toward the ventral side, the thumb side, or the nail side of the fingertip.

The manual muscle strength testing device of the invention according to claim 3, in addition to the effect of the invention according to claim 1 or 2, including the pressure sensor wearable on the fingertip, being capable of measuring magnitude of the load applied to either the ventral side, the thumb side, or the nail side of the fingertip and recording or transmitting load measurement data, wherein
the inertial sensor and the pressure sensor are fixed to the ring wearable on the fingertip,
the pressure sensor is fixed to all of or any one of the ventral side, the thumb side, and the nail side of the fingertip at the outside circular portion of the ring, and
the load applying portion applies the load to the pressure sensor fixed to either the ventral side, the thumb side, or the nail side of the fingertip. Therefore, the manual muscle strength testing device is able to measure the magnitude of the load applied on the fingertip when the index finger moves.

### Brief Description of Drawings

FIG. 1 is a perspective view and a side view illustrating a manual muscle strength testing device and a first use example according to Example 1.
FIG. 2 is a perspective view of the main body of the manual muscle strength testing device according to Example 1.
FIG. 3 is perspective views of a ring, a finger press, an other-finger press, and a fingerstall used in Example 1.
FIG. 4 is a perspective view and a side view illustrating the manual muscle strength testing device and a second use example according to Example 1.
FIG. 5 is a perspective view and a side view illustrating the manual muscle strength testing device and a third use example according to Example 1.
FIG. 6 is a diagram illustrating the relationship between a displacement amount and amplitude δ based on an output of an acceleration sensor.
FIG. 7 is graphs illustrating δa values of a median nerve obtained by the first use example.
FIG. 8 is graphs illustrating δa values of an ulnar nerve obtained by the second use example.
FIG. 9 is graphs illustrating δa values of a radial nerve obtained by the third use example.
FIG. 10 is a perspective view illustrating a structure of the manual muscle strength testing device according to Example 2.
FIG. 11 is diagrams illustrating appearances of a ring worn on an index finger and the ring in Example 2.
FIG. 12 is diagrams illustrating the first to third use examples of the manual muscle strength testing device according to Example 2.
FIG. 13 is perspective views illustrating a structure of a finger insertion portion in the manual muscle strength testing device according to Example 3.
FIG. 14 is diagrams illustrating the first to sixth use examples of the manual muscle strength testing device according to Example 3.

### Description of Embodiments

Embodiments of the present invention will now be described with reference to Examples.

### [Example 1]

FIG. 1 is a perspective view and a side view illustrating a manual muscle strength testing device and a first use example according to Example 1, and FIG. 2 is a perspective view of the main body of the manual muscle strength testing device according to Example 1.

As illustrated in FIG. 1(a), FIG. 1(b), and FIG. 2, the manual muscle strength testing device according to Example 1 includes the following configurations (A) to (I). (A) A palm rest 1 including a rectangular plate on which an entire palm P of a subject can be placed.

The plate of the palm rest 1 is provided with a plurality of holes 2 in a matrix form, and the two holes 2 near the center of the long side are connected through a slit 3. A pin 4 is fixed in each of two rows × seven holes 2 in the center, and the head of each of the pin 4 protrudes from the bottom face of the plate. (B) A side wall 5 having a rectangular shape extending downward from one side of the palm rest 1.

The side wall 5 is provided with an opening 6 and legs 7, the opening 6 being formed in the center, the legs 7 being disposed between the left and right sides and extending from peripheral parts on the bottom face of the palm rest 1. Also, from another peripheral part on the bottom face of the palm rest 1, a leg 7 extends. The palm rest 1 and the side wall 5 are supported by these three legs 7.

Although three legs 7 are used in order to improve workability on the bottom face side of the palm rest 1, thin legs may be extended from the peripheral part on the bottom face provided with no leg 7 to increase stability.

Conversely, the legs 7 need not be provided inside the side wall 5. Furthermore, without the legs 7, a rectangular auxiliary member having approximately the same height as the side wall 5 may be placed opposite to the side wall 5 and the palm rest 1 may be placed on the top of the auxiliary member to be used. (C) A restrainer 8 configured to fix the palm P to the top face of the palm rest 1.

The restrainer 8 has a buckle 10 at one end of a flat belt 9, and the other end of the flat belt 9 is passed through the buckle 10 and then folded back to adjust the length.

That is, the other end of the flat belt 9 is passed through the slit 3 of the front side in FIG. 1, and then passed through the slit 3 of the back side in FIG. 1 from the bottom face, then set on the buckle 10 over the palm P of the subject to adjust the length of the flat belt 9, as illustrated in FIG. 1(a), and thereby the palm P can be fixed to the top face of the palm rest 1. (D) A three-axis acceleration sensor 11 configured to measure movement of a fingertip and transmit measurement data.

The three-axis acceleration sensor 11 is worn on at least one fingertip (index finger in FIG. 1(a)) of the palm P of the subject, measures the movement of the fingertip, and transmits the measurement data.

When the measurement data transmitted from the three-axis acceleration sensor 11 is received to be processed and analyzed by an analysis unit (not illustrated), the amplitude δ of acceleration data in the movable direction of the fingertip can be measured.

The measurement data need not be transmitted from the three-axis acceleration sensor 11, but may be recorded in a memory together with the measurement time and may be retrieved by connecting the memory to the analysis unit after the measurement is completed. (E) A load applying portion 12 configured to apply an adjustable load to the fingertip to be measured for movement.

The load applying portion 12 includes binding bands 13 set in through holes 19 of a ring 15 described later, two rubber bands 14, and the pins 4 (an end of the rubber band 14 can be hooked on the head) fixed in the two rows × seven holes 2 described in (A) above.

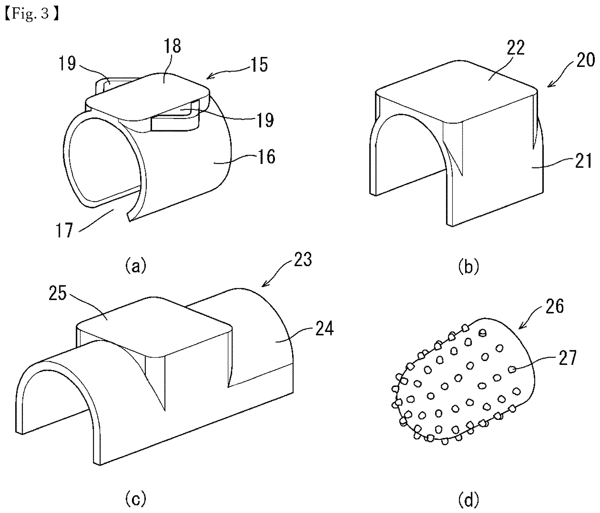

FIGS. 3(a) to 3(d) are perspective views of the ring 15, a finger press 20, an other-finger press 23, and a fingerstall 26 used in Example 1, and each configuration will be described below. (F) The ring 15 configured to fit to the fingertip of a subject and fix the three-axis acceleration sensor 11.

As illustrated in FIG. 3(a), the ring 15 includes a circular portion 16 into which the fingertip (index finger in FIG. 1(a)) of the subject can be inserted, a sensor fixation portion 18 provided at a position opposite to a cut 17 of the circular portion 16, and through holes 19 provided at both sides of the sensor fixation portion 18.

The binding bands 13 are set in the through holes 19 to be used during measurement.

The circular portion 16 is provided with the cut 17, and the circular portion 16 itself has elasticity. It is thus possible to fit the fingertip into the circular portion 16 even if the finger thickness differs by a few millimeters. However, since the finger thickness varies greatly for individuals and the ring 15 may be fit to a fingertip other than the index finger to be used, it is better to be available several types of the rings 15 with different diameters of the circular portion 16.

It is preferable that the diameter of the circular portion 16 be smaller at the tip end side and larger at the base end side. (G) The finger press 20 configured to press the finger to be measured for movement, toward the palm rest 1.

The finger press 20 is designed to press the part between the second and third joints of the finger to be measured for movement (index finger in FIG. 1(a)), the finger wearing the three-axis acceleration sensor 11, toward the palm rest 1 in a state where the finger is fixed to the top face of the palm rest 1 with the palm P of the subject facing up or down. As illustrated in FIG. 3(b), the finger press 20 includes a U-shaped portion 21 that is high enough that the lower end contacts the top face of the palm rest 1 when the finger press 20 covers and presses the part between the second and third joints of the finger to be measured, and a finger press flat portion 22 provided at an upper side of the U-shaped portion 21.

As with the ring 15, it is preferable that the finger press 20 also be available in several types with different widths and heights of the U-shaped portion 21. (H) The other-finger press 23 configured to press a finger adjacent to the finger to be measured for movement, toward the palm rest 1.

The other-finger press 23 is designed to press the finger adjacent to the finger wearing the three-axis acceleration sensor 11, toward the palm rest 1 in a state where the finger adjacent to the finger to be measured for movement (middle finger in FIG. 1(a)) is fixed to the top face of the palm rest 1 with the palm P of the subject facing upward. As illustrated in FIG. 3(c), the other-finger press 23 includes a semicircular portion 24 that is high enough that the lower end contacts the top face of the palm rest 1 when the other-finger press 23 covers and presses the finger to be pressed, and an other-finger press flat portion 25 provided at the center of the upper side of the semicircular portion 24.

As with the ring 15 and the finger press 20, it is preferable that the other-finger press 23 also be available in several types with different widths and heights of the semicircular portion 24. (I) The fingerstall 26 configured to fit to the fingertip of the subject and stabilize the position of the ring 15.

The fingerstall 26 is designed to fit the fingertip of the finger to be measured for movement (index finger in FIG. 1(a)) to stabilize the position of the ring 15 and to improve wearability of the ring 15. As illustrated in FIG. 3(d), the fingerstall 26 is formed by molding an elastic material such as rubber into a dome shape with a closed tip end side and numerous protrusions 27 on the surface.

As with the ring 15 and the like, it is preferable that the fingerstall 26 also be available in several types with different diameters and sizes, and it is more preferable that ventilation holes be provided on the side face to prevent steaming.

In the first use example, as illustrated in FIG. 1, by using the manual muscle strength testing device, the palm P of the subject is turned upward and fixed to the top face of the palm rest 1, and the movement of the fingertip is measured while instructions are given to the subject such that the index finger of the subject is bent at the second joint to move the fingertip upward and then returned to the original position. The state of flexor digitorum superficialis can thus be determined. Furthermore, by analyzing the measurement values, the state and the recovery level of the median nerve can be evaluated.

The procedure of the measurement in the first use example will be described in detail.

(Procedure A1) The fingerstall 26 is worn on the fingertip of the index finger of the subject.
(Procedure A2) The ring 15 matching the size of the fingertip wearing the fingerstall 26 is selected, and the three-axis acceleration sensor 11 is fixed to the sensor fixation portion 18 with a double-sided tape or an adhesive.

If the ring 15 with the three-axis acceleration sensor 11 fixed to the sensor fixation portion 18 is selected, the fixation work of the three-axis acceleration sensor 11 is not necessary.
(Procedure A3) The index finger of the subject wearing the fingerstall 26 is inserted into the circular portion 16 of the ring 15 to which the three-axis acceleration sensor 11 is fixed, the circular portion 16 is tightly fit between the first joint and the fingertip such that the upper face of the three-axis acceleration sensor 11 and the ventral side of the fingertip face the same direction.
(Procedure A4) After the fixation of the three-axis acceleration sensor 11, the palm P of the subject is turned upward and placed on the top face of the palm rest 1. The palm P is fixed to the top face of the palm rest 1 by passing the other end of the flat belt 9 through one slit 3 and then through the other slit 3 from the bottom face of the palm rest 1, setting the other end on the buckle 10 over the center of the palm P of the subject, and then adjusting the length of the flat belt 9.

The back of the hand of the subject may be pressed and fixed to the palm rest 1 with a hand H of an examiner without use of the restrainer 8 for fixing the palm P to the top face of the palm rest 1.

(Procedure A5) One end of one rubber band 14 is hooked on each of the binding bands 13 set on both sides of the ring 15, the other end of the rubber band 14 is passed through the hole 2 in the palm rest 1 and hooked on any pin 4 in one row (the second pin 4 from the arm side in FIG. 1(b)). The other end of the other rubber band 14 is similarly hooked on the pin 4 at the corresponding position in the other row through the hole 2 in the palm rest 1.

Here, a state in which the other ends of the two rubber bands 14 are not hooked on the pins 4 is referred to as load 0, a state in which the other ends of the two rubber bands 14 are hooked on the pins 4 positioned where the rubber bands 14 are most contracted (hereinafter referred to as "shortest position") is referred to as load 1, a state in which the other ends of the two rubber bands 14 are hooked on the pins 4 placed at one position closer to the arm side from the shortest position is referred to as load 2, a state in which the other ends of the two rubber bands 14 are hooked on the pins 4 placed at two positions closer to the arm side from the shortest position is referred to as load 3, and a state in which the other ends of the two rubber bands 14 are hooked on the pins 4 placed at three positions closer to the arm side from the shortest position is referred to as load 4. (Procedure A6) The finger press 20 is caused to cover and press the part between the second and third joints of the index finger toward the palm rest 1, and the other-finger press 23 is caused to cover and press the middle finger toward the palm rest 1.

The method of the pressing can be selected from a variety of ways, such as with the hand of the examiner, by placing a weight on the finger press flat portion 22 and the other-finger press flat portion 25, or by fixing the finger press 20 and the other-finger press flat portion 25 to the top face of the palm rest 1 using an elastic material such as a rubber string or a fitting member to the hole 2. (Procedure A7) The three-axis acceleration sensor 11 is activated, and instructions are given to the subject such that the index finger is bent at the second joint for three seconds, held in a maximum movable range for three seconds, and then returned to the original position for three seconds. These instructions are repeated five times to measure the movement of the fingertip. After the measurement is completed, the three-axis acceleration sensor 11 is stopped.

FIG. 4 is a perspective view and a side view illustrating the manual muscle strength testing device and a second use example according to Example 1.

In the second use example, as illustrated in FIG. 4, the middle, ring, and little fingers are fixed to the top face of the palm rest 1 with the palm P of the subject turned sideways, and then the movement of the fingertip is measured while instructions are given to the subject such that the entire index finger of the subject is opened against the middle finger, the fingertip is moved upward, and the index finger is then returned to the original position by using the manual muscle strength testing device in Example 1. The state of dorsal interossei can thus be determined. Furthermore, by analyzing the measurement values, the state and the recovery level of the ulnar nerve can be evaluated.

The procedure of the measurement in the second use example will be described in detail.

(Procedure B1) The ring 15 matching the size of the index finger of the subject is selected, and the three-axis acceleration sensor 11 is fixed to the sensor fixation portion 18 with a double-sided tape or an adhesive.

If the ring 15 with the three-axis acceleration sensor 11 fixed to the sensor fixation portion 18 is selected, the fixation work of the three-axis acceleration sensor 11 is not necessary.

The fingerstall 26 may be worn on the fingertip of the index finger of the subject, as in the procedure A1. In such case, the ring 15 matching the size of the fingertip wearing the fingerstall 26 is selected.

(Procedure B2) The index finger of the subject is inserted into the circular portion 16 of the ring 15 to which the three-axis acceleration sensor 11 is fixed, and the circular portion 16 is tightly fit and fixed between the first joint and the fingertip such that the upper face of the three-axis acceleration sensor 11 and the thumb side of the fingertip face the same direction.
(Procedure B3) After the fixation of the three-axis acceleration sensor 11, the palm P of the subject is turned sideways and placed on the top face of the palm rest 1, and the middle, ring, and little fingers are fixed to the top face of the palm rest 1.

The middle, ring, and little fingers may be fixed with the hand H of the examiner as illustrated in FIG. 4(a), or may be fixed by providing slits similar to the slit 3 at appropriate positions in the palm rest 1, passing the other end of the flat belt 9 of the restrainer 8 through the two slits as in the procedure A4, setting the other end on the buckle 10 around the ring finger of the palm P of the subject, and then adjusting the length of the flat belt 9.
(Procedure B4) One end of one rubber band 14 is hooked on each of the binding bands 13 set on both sides of the ring 15, the other end of the rubber band 14 is passed through the hole 2 in the palm rest 1 and hooked on any pin 4 in one row (positioned second from the side of the fingertip in FIG. 4(b)). The other end of the other rubber band 14 is hooked on the pin 4 at the corresponding position in the other row through the hole 2 in the palm rest 1.

The definitions of loads 0 to 3 are the same as described in the procedure A5.
(Procedure B5) After the two rubber bands 14 are hooked on the pins 4, the three-axis acceleration sensor 11 is activated, and instructions are given to the subject such that the entire index finger is opened against the middle finger for three seconds, held in the maximum movable range for three seconds, and then returned to the original position for three seconds. These instructions are repeated five times to measure the movement of the fingertip. After the measurement is completed, the three-axis acceleration sensor 11 is stopped.

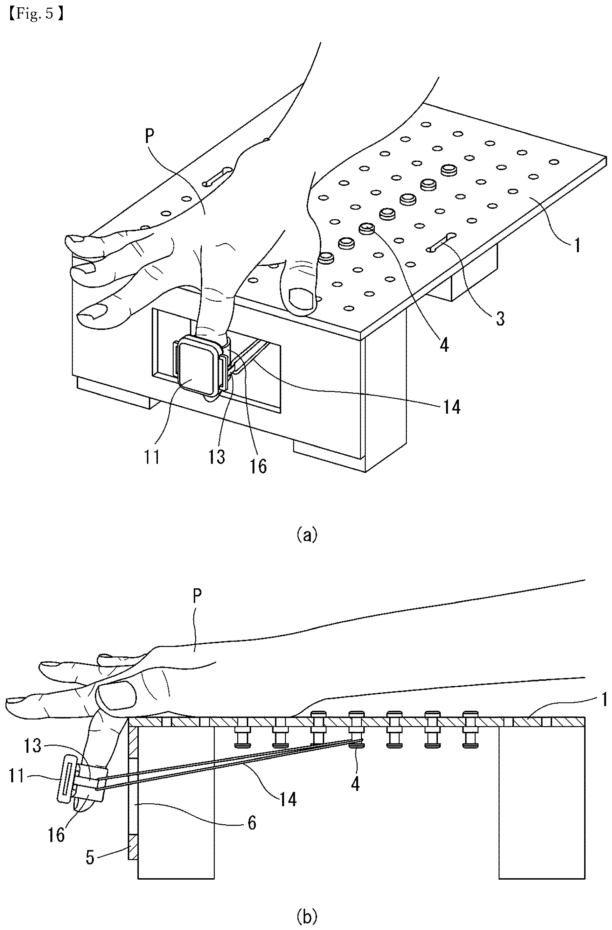

FIG. 5 is a perspective view and a side view illustrating the manual muscle strength testing device and a third use example according to Example 1.

In the third use example, as illustrated in FIG. 5, the palm P of the subject is turned downward and fixed to the top face of the palm rest 1 with the second joint of the index finger positioned slightly in front of the side wall 5, and the movement of the fingertip is measured while instructions are given to the subject such that the fingertip of the index finger of the subject is extended from a state of 90-degree bent at the second joint and then the finger is returned to the original position by using the manual muscle strength testing device in Example 1. The state of extensor digitorum can thus be determined. Furthermore, by analyzing the measurement values, the state and the recovery level of the radial nerve can be evaluated.

The procedure of the measurement in the third use example will be described in detail.
(Procedure C1) The ring 15 matching the size of the index finger of the subject is selected, and the three-axis acceleration sensor 11 is fixed to the sensor fixation portion 18 with a double-sided tape or an adhesive.

If the ring 15 with the three-axis acceleration sensor 11 fixed to the sensor fixation portion 18 is selected, the fixation work of the three-axis acceleration sensor 11 is not necessary.

The fingerstall 26 may be worn on the fingertip of the index finger of the subject, as in the procedure A1. In such case, the ring 15 matching the size of the fingertip wearing the fingerstall 26 is selected.
(Procedure C2) The index finger of the subject is inserted into the circular portion 16 of the ring 15 to which the three-axis acceleration sensor 11 is fixed, and the circular portion 16 is tightly fit and fixed between the first joint and the fingertip such that the upper face of the three-axis acceleration sensor 11 and the nail side of the fingertip face the same direction.
(Procedure C3) After the fixation of the three-axis acceleration sensor 11, the palm P of the subject is turned downward and placed on the top face of the palm rest 1 and is fixed to the palm rest 1.

The palm P may be fixed with the hand of the examiner as in the procedure B3 described above, or may be fixed by providing slits similar to the slit 3 at appropriate positions in the palm rest 1, passing the other end of the flat belt 9 of the restrainer 8 through the two slits as in the procedure A4, setting the other end on the buckle 10 around the third joint of the middle finger of the subject, and then adjusting the length of the flat belt 9.
(Procedure C4) One end of one rubber band 14 is hooked on each of the binding bands 13 set on both sides of the ring 15, the other end of the rubber band 14 is passed through the opening 6 of the side wall 5 and hooked on any pin 4 in one row (positioned fourth from the side of the fingertip in FIG. 5(b)). The other end of the other rubber band 14 is hooked on the pin 4 at the corresponding position in the other row through the opening 6 of the side wall 5.

The definitions of loads 0 to 3 are the same as described in the procedure A5.

After the rubber bands 14 are hooked on the pins 4, the finger press 20 may be caused to cover and press the part between the second and third joints of the index finger toward the palm rest 1 as in the procedure A6.
(Procedure C5) After the two rubber bands 14 are hooked on the pins 4, the three-axis acceleration sensor 11 is activated, and instructions are given to the subject such that the index finger is extended for three seconds from a state of 90-degree bent at the second joint, held in the maximum movable range for three seconds, and then returned to the original position for three seconds. These instructions are repeated five times to measure the movement of the fingertip. After the measurement is completed, the three-axis acceleration sensor 11 is stopped.

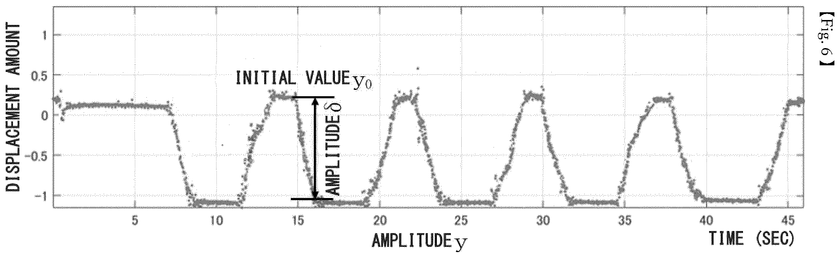

FIG. 6 is a diagram illustrating the relationship between a displacement amount and amplitude δ based on an output of the acceleration sensor 11.

As described above, according to the procedures A1 to A7 of the first use example, the palm P of the subject is turned upward and fixed to the top face of the palm rest 1, and the movement of the fingertip can be measured when the index finger is bent and extended at the second joint. According to the procedures B1 to B6 of the second use example, the middle, ring, and little fingers are fixed to the top face of the palm rest 1 with the palm P of the subject turned sideways, and the movement of the fingertip can be measured when the entire index finger is opened against the middle finger. According to the procedures C1 to C6 of the third use example, the palm P of the subject is turned downward and fixed to the top face of the palm rest 1, and the movement of the fingertip can be measured when the index finger is extended from a state of 90-degree bent at the second joint and bent again. By analyzing the measurement values, the state and the recovery level of the median, ulnar, and radial nerves can be evaluated.

Although the amplitude differs in each of the first to third use examples, a graph of the movement of the fingertip based on measurement data transmitted from the acceleration sensor 11, with the horizontal axis representing a time axis and the vertical axis representing a displacement amount in the Y-axis direction, has the shape illustrated in FIG. 6.

In the analysis of the measurement data obtained by the first to third use examples, the position of the fingertip before movement was referred to as initial value y₀, the position where the fingertip was moved and held in the maximum movable range for three seconds was referred to as holding value y, and the state and the recovery level of the median, ulnar, and radial nerves were evaluated by calculating the difference between the initial value y₀ and the holding value y (y₀-y) to measure the amplitude δ, and calculating the average value δa of the amplitude δ for a total of five times.

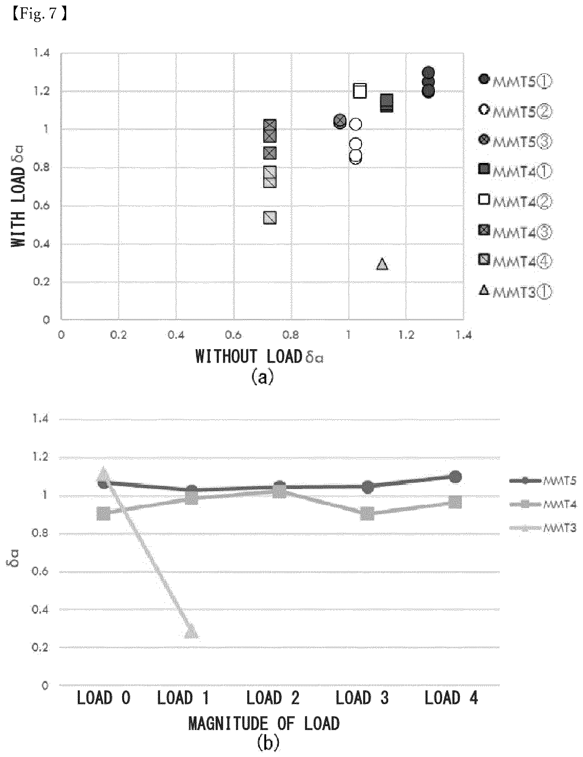

FIG. 7 is graphs illustrating δa values of a median nerve obtained by the first use example.

FIG. 7(a) plots the variance of δa at load 0 and δa at loads 1 to 4 for each subject, with the horizontal axis of the graph representing δa without any load and the vertical axis representing δa with a load.

The subjects plotted in circles are subjects who have been evaluated in advance as MMT5 (Normal) by a physician, the subjects plotted in squares are subjects who have been evaluated as MMT4 (Good), and the subjects plotted in triangles are subjects who have been evaluated as MMT3 (Fair).

FIG. 7(b) is a graph comparing transitions of the approximate lines of δa at loads 0 to 4 by the group of subjects who have been evaluated in advance as MMT3 to MMT5 by a physician.

These graphs indicate that the group of subjects with MMT5 has δa often exceeding 1 at any of loads 0 to 4 and is plotted in the upper right region of FIG. 7(a), the group of subjects with MMT4 has δa close to 1 at loads 0 to 4 but varies between the subjects close to MMT5 and the subjects close to MMT3 according to FIG. 7(a), and the subject with MMT3 (one subject) is able to move the fingertip to some extent at load 1 and is thus in the same characteristic state as the criteria for MMT3 (a state where a force sufficient to move the joint is exerted against the gravity).

Based on the above findings, when the variance of δa at load 0 and δa at loads 1 to 4 are plotted on the graph in FIG. 7(a), if plotted in the upper right region, the subject is likely to belong to the group of MMT5, if plotted between the upper right and lower left regions, the subject is likely to belong to the group of MMT4, and if plotted in the lower left region, the subject is likely to belong to the group of MMT3.

Furthermore, the group of MMT4 has wide variation and is difficult to determine for physicians. However, it is possible to determine whether the state is closer to the group of MMT5 or the group of MMT3 by plotting the variance of δa at load 0 and δa at loads 1 to 4 on the graph in FIG. 7(a). This can thus provide a more detailed evaluation than the conventional 6 steps evaluation.

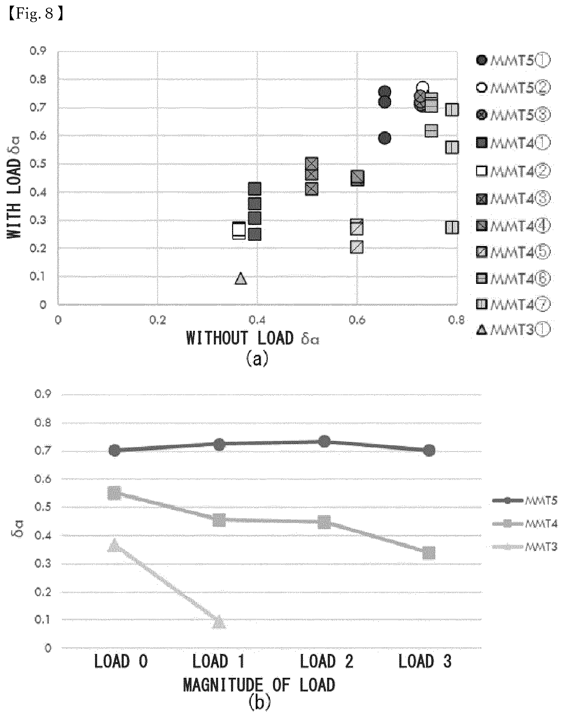

FIG. 8 is graphs illustrating δa values of the ulnar nerve obtained by the second use example.

FIG. 8(a) plots the variance of δa at load 0 and δa at loads 1 to 3 for each subject, with the horizontal axis of the graph representing δa without any load and the vertical axis representing δa with a load.

The circle, square, and triangle plots, as in FIG. 7(a), illustrate the subjects evaluated as MMT5, MMT4, and MMT3 in advance by a physician, respectively.

FIG. 8(b) is a graph comparing transitions of the approximate lines of δa at loads 0 to 3 by the group of subjects who have been evaluated in advance as MMT3 to MMT5 by a physician.

These graphs indicate that the group of subjects with MMT5 has δa often exceeding 0.7 at any of loads 0 to 3 and is plotted in the upper right region of FIG. 8(a), the group of subjects with MMT4 often has δa in the range of 0.4 to 0.6 at loads 0 to 3 but varies widely from the subject close to MMT5 to the subject close to MMT3 according to FIG. 8(a), and the subject with MMT3 (one subject) is able to move the fingertip to some extent at load 1 and is thus in the same characteristic state as the criteria for MMT3 (the state where a force sufficient to move the joint is exerted against the gravity).

Based on the above findings, when the variance of δa at load 0 and δa at loads 1 to 3 are plotted on the graph in FIG. 8(a), if plotted in the upper right region, the subject is likely to belong to the group of MMT5, if plotted between the upper right and lower left regions, the subject is likely to belong to the group of MMT4, and if plotted in the lower left region, the subject is likely to belong to the group of MMT3.

It is also clear that in the subjects with MMT4 and MMT3, δa values decrease with an increase in the loads.

Furthermore, the group of MMT4 has wide variation and is difficult to determine for physicians as in FIG. 7. However, it is possible to determine whether the state is closer to the group of MMT5 or the group of MMT3 by plotting the variance of δa at load 0 and δa at loads 1 to 3 on the graph in FIG. 8(a). This can thus provide a more detailed evaluation than the conventional 6 steps evaluation.

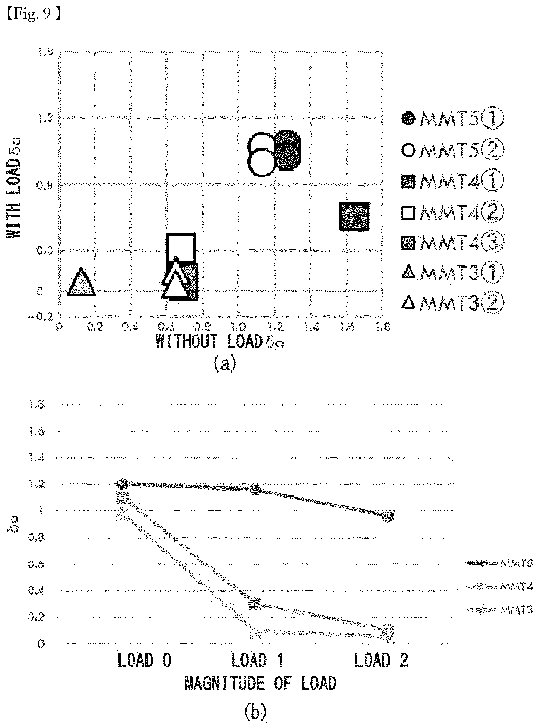

FIG. 9 is graphs illustrating δa values of a radial nerve obtained by the third use example.

FIG. 9(a) plots the variance of δa at load 0 and δa at loads 1 to 2 for each subject, with the horizontal axis of the graph representing δa without any load and the vertical axis representing δa with a load.

The circle, square, and triangle plots, as in FIG. 7(a), illustrate the subjects evaluated as MMT5, MMT4, and MMT3 in advance by a physician, respectively.

FIG. 9(b) is a graph comparing transitions of the approximate lines of δa at loads 0 to 2 by the group of subjects who have been evaluated in advance as MMT3 to MMT5 by a physician.

These graphs indicate that the group of subjects with MMT5 often has δa around 1.2 at any of loads 0 to 1, around 1 at load 2, and is plotted in the upper right region of FIG. 9(a), the group of subjects with MMT4 and MMT3 has δa around 1 at load 0 but often has δa dropping sharply from 0.4 to 0.1 at loads 1 to 2, and has lower δa with a load than the group of subjects with MMT5 according to FIG. 9(a). It can be seen that the subject with MMT3 has lower δa than the group of MMT4 at any of loads 0 to 2.

Based on the above findings, when the variance of δa at load 0 and δa at loads 1 to 3 are plotted on the graph in FIG. 9(a), if plotted in the upper right region, the subject is likely to belong to the group of MMT5, if plotted in the lower center to lower right region, the subject is likely to belong to the group of MMT4, and if plotted in the lower left region, the subject is likely to belong to the group of MMT3.

Furthermore, it is considered that a smaller load should be applied to obtain the δa values when the recovery level of the radial nerve is evaluated in the third use example, which indicates that the way the load is applied is important.

### [Example 2]

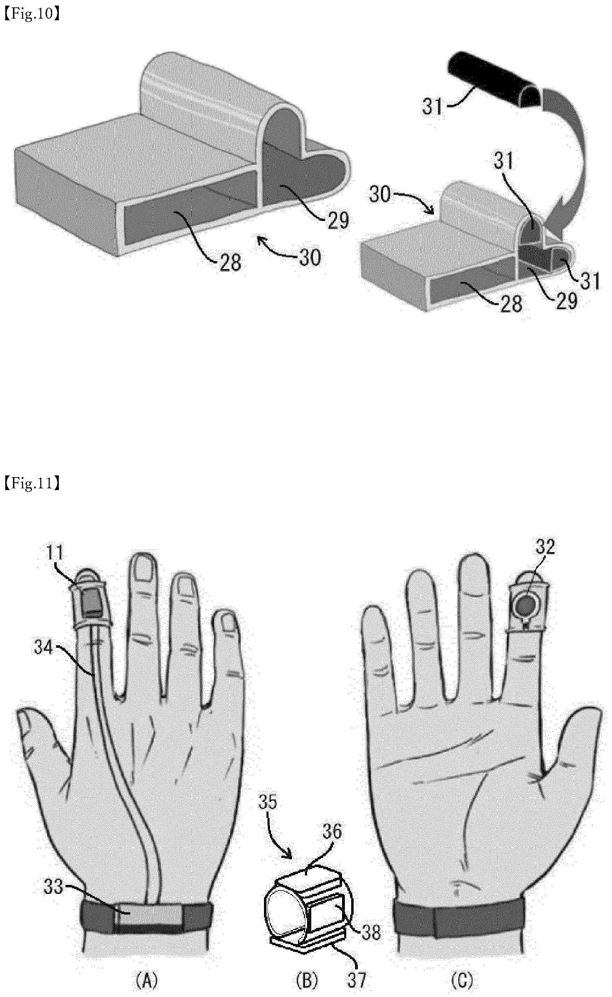

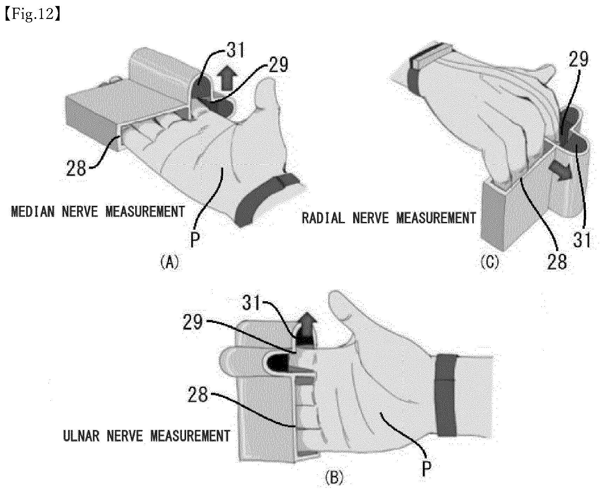

FIG. 10 is a perspective view illustrating a structure of the manual muscle strength testing device according to Example 2, FIG. 11 is diagrams illustrating appearances of a ring worn on an index finger and the ring in Example 2, and FIG. 12 is diagrams illustrating the first to third use examples of the manual muscle strength testing device according to Example 2.

As illustrated in FIGS. 10 to 12, the manual muscle strength testing device according to Example 2 includes the following configurations (J) to (N). Some configurations are common to configurations in Example 1. The same numbers may be used and the explanation may be simplified for the common configurations.

(J) A finger insertion portion 30 that includes an other-finger fixation portion 28 having a space shaped such that the middle, ring, and little fingers of a subject can be inserted and the inserted fingers cannot move in any direction, and an index finger insertion portion 29 having a space shaped such that the index finger can be inserted and the inserted index finger can move to the ventral or thumb side, or the nail or thumb side.

The other-finger fixation portion 28 has a square tubular shape into which the middle, ring, and little fingers can be inserted from both sides, and the index finger insertion portion 29 has an L-shaped tubular cross section into which the index finger can be inserted from both sides.

Since the size of the fingers varies for individuals, a plurality of the finger insertion portions 30 with different lengths and heights of the other-finger fixation portion 28 are prepared in advance, especially in consideration of the width and thickness of the middle, ring, and little fingers when they are aligned.
(K) A load elastic body 31 that can be inserted into, fixed to and removed from a protruding portion of the index finger insertion portion 29.

The load elastic body 31 corresponds to the load applying portion 12 (especially the two rubber bands 14) in Example 1. For example, by using elastic bodies with various softness (a sponge in FIG. 10), an adjustable load can be applied to the fingertip.

In FIG. 10, the protruding portion of the index finger insertion portion 29 has an arch-shaped cross section, the load elastic body 31 therefore also has a semi-elliptical shape. However, the protruding portion of the index finger insertion portion 29 may have a U-shaped cross section with right-angled corners. In such case, the load elastic body 31 has a prismatic shape.
(L) The three-axis acceleration sensor 11 configured to measure the movement of the fingertip and transmit the measurement data.

For details on the three-axis acceleration sensor 11, see (D) in Example 1.
(M) A pressure sensor 32 configured to measure magnitude of the load applied to the fingertip and transmit the measurement data.

In FIG. 11(C), the pressure sensor 32 is fixed to the ventral side of the index finger of the subject. When the fingertip of the index finger is moved to the ventral side inside the index finger insertion portion 29, as in FIG. 12(A) described later, the pressure sensor 32 receives a repulsive force from the load elastic body 31.

When pressure measurement data measured by the pressure sensor 32 is received, processed, and analyzed by an analysis unit (not illustrated), the magnitude of the pressure applied to the ventral side of the fingertip can be determined.

In Example 2, in order to transmit acceleration measurement data and the pressure measurement data measured by the three-axis acceleration sensor 11 and the pressure sensor 32, as illustrated in FIG. 11(A), a data transmission instrument 33 is worn on the wrist of the subject, and the three-axis acceleration sensor 11, the pressure sensor 32, and the data transmission instrument 33 are connected with signal transmission lines 34.
(N) A ring 35 configured to fix the three-axis acceleration sensor 11 and the pressure sensor 32 to the fingertip.

As illustrated in FIG. 11(B), the ring 35, as with the ring 15 described in (F) in Example 1, includes a circular portion into which the fingertip of the index finger of the subject can be inserted, and an upper face sensor fixation portion 36 provided at a position opposite to a cut in the circular portion. The ring 35 further includes a lower face sensor fixation portion 37 firmly fixed to one side of the cut in the circular portion (left side in the drawing), and a side face sensor fixation portion 38 firmly fixed to one side of the circular portion (right side in the drawing).

In the first and third use examples described later, the three-axis acceleration sensor 11 is fixed to one of the upper face sensor fixation portion 36 and the lower face sensor fixation portion 37, and the pressure sensor 32 is fixed to the other to measure the movement of the fingertip and the pressure applied to the ventral side of the fingertip in the first use example or the pressure applied to the nail side of the fingertip in the third use example. In the second use example described later, the three-axis acceleration sensor 11 is fixed to either the upper face sensor fixation portion 36 or the lower face sensor fixation portion 37, and the pressure sensor 32 is fixed to the side face sensor fixation portion 38 to measure the movement of the fingertip and the pressure applied to the thumb side of the fingertip.

To stabilize the position of the ring 35, the fingerstall 26 described in (I) in Example 1 and illustrated in FIG. 3(d) may be used.

FIG. 12(A) is a diagram illustrating the first use example of the manual muscle strength testing device according to Example 2.

In the first use example in Example 2, after the load elastic body 31 with appropriate softness is inserted into and fixed to the protruding portion of the index finger insertion portion 29, the palm P of the subject is turned upward, the middle, ring, and little fingers are inserted into the other-finger fixation portion 28 and the index finger is inserted into the index finger insertion portion 29.

The state of the flexor digitorum superficialis can then be determined by measuring the movement of the fingertip and the pressure applied to the ventral side of the fingertip while instructions are given to the subject such that the index finger is bent at the second joint to move the fingertip upward (in the direction of the arrow) and then returned to the original position. The state and the recovery level of the median nerve can be evaluated as in Example 1 by analyzing the measurement values.

With regard to procedures corresponding to the procedures A1 to A4 in the first use example in Example 1, for the first use example in Example 2, the procedure is completed simply by wearing the ring 35, to which the three-axis acceleration sensor 11 and the pressure sensor 32 are fixed, on the index finger of the subject, inserting the middle, ring, and little fingers into the other-finger fixation portion 28 and inserting the index finger into the index finger insertion portion 29, which is simpler than the procedure in Example 1.

With regard to a procedure corresponding to the procedure A5 in the first use example in Example 1, for the first use example in Example 2, a state in which no load elastic body 31 is inserted is load 0, a state in which the softest load elastic body 31 is inserted and fixed is load 1, a state in which the second softest load elastic body 31 is inserted and fixed is load 2, a state in which the third softest load elastic body 31 is inserted and fixed is load 3, and a state in which the fourth softest (hardest) load elastic body 31 is inserted and fixed is load 4.

Furthermore, with regard to procedures corresponding to the procedures A6 and A7 in the first use example in Example 1, for the first use example in Example 2, the procedure A6 can be omitted, the three-axis acceleration sensor 11 and the pressure sensor 32 are activated, instructions are given to the subject such that the index finger is bent at the second joint for three seconds, held in a maximum movable range for three seconds, and then returned to the original position for three seconds. The movement of the fingertip and the pressure applied to the ventral side of the fingertip are measured while the instructions are repeated five times, the three-axis acceleration sensor 11 and the pressure sensor 32 are stopped after the measurement, and this procedure is thus almost the same as the procedure A7 in Example 1.

FIG. 12(B) is a diagram illustrating the second use example of the manual muscle strength testing device according to Example 2.

In the second use example in Example 2, after the load elastic body 31 with appropriate softness is inserted into and fixed to the protruding portion of the index finger insertion portion 29, the palm P of the subject is turned sideways, the middle, ring, and little fingers are inserted into the other-finger fixation portion 28 and the index finger is inserted into the index finger insertion portion 29.

The state of the dorsal interossei can then be determined by measuring the movement of the fingertip and the pressure applied to the thumb side of the fingertip while instructions are given to the subject such that the entire index finger is opened against the middle finger, the fingertip is moved upward (in the direction of the arrow), and then returned to the original position. The state and the recovery level of the ulnar nerve can be evaluated as in Example 1 by analyzing the measurement values.

With regard to procedures corresponding to the procedures B1 to B3 in the second use example in Example 1, for the second use example in Example 2, the procedure is completed simply by wearing the ring 35, to which the three-axis acceleration sensor 11 and the pressure sensor 32 are fixed, on the index finger of the subject, inserting the middle, ring, and little fingers into the other-finger fixation portion 28 and inserting the index finger into the index finger insertion portion 29, which is simpler than the procedure in Example 1.

With regard to a procedure corresponding to the procedure B4 in the second use example in Example 1, for the second use example in Example 2, the state in which no load elastic body 31 is inserted is load 0, the state in which the softest load elastic body 31 is inserted and fixed is load 1, the state in which the second softest load elastic body 31 is inserted and fixed is load 2, the state in which the third softest load elastic body 31 is inserted and fixed is load 3, and the state in which the fourth softest (hardest) load elastic body 31 is inserted and fixed is load 4, as in the first use example in Example 2.

Furthermore, with regard to a procedure corresponding to the procedure B5 in the second use example in Example 1, for the second use example in Example 2, the three-axis acceleration sensor 11 and the pressure sensor 32 are activated, instructions are given to the subject such that the entire index finger is opened against the middle finger for three seconds, held in a maximum movable range for three seconds, and then returned to the original position for three seconds. The movement of the fingertip and the pressure applied to the thumb side of the fingertip are measured while the instructions are repeated five times, the three-axis acceleration sensor 11 and the pressure sensor 32 are stopped after the measurement, and this procedure is thus almost the same as the procedure B5 in Example 1.

FIG. 12(C) is a diagram illustrating the third use example of the manual muscle strength testing device according to Example 2.

In the third use example in Example 2, after the load elastic body 31 with appropriate softness is inserted into and fixed to the protruding portion of the index finger insertion portion 29, the palm P of the subject is turned downward, the middle, ring, and little fingers are inserted into the other-finger fixation portion 28 and the index finger is inserted into the index finger insertion portion 29.

The state of the extensor digitorum can then be determined by measuring the movement of the fingertip and the pressure applied to the nail side of the fingertip while instructions are given to the subject such that the fingertip is extended in the arrow direction from a state of 90-degree bent at the second joint and then returned to the original position. The state and the recovery level of the radial nerve can be evaluated as in Example 1 by analyzing the measurement values.

With regard to procedures corresponding to the procedures C1 to C3 in the third use example in Example 1, for the third use example in Example 2, the procedure is completed simply by wearing the ring 35, to which the three-axis acceleration sensor 11 and the pressure sensor 32 are fixed, on the index finger of the subject, inserting the middle, ring, and little fingers into the other-finger fixation portion 28 and inserting the index finger into the index finger insertion portion 29, which is simpler than the procedure in Example 1.

With regard to a procedure corresponding to the procedure C4 in the third use example in Example 1, for the third use example in Example 2, the state in which no load elastic body 31 is inserted is load 0, the state in which the softest load elastic body 31 is inserted and fixed is load 1, the state in which the second softest load elastic body 31 is inserted and fixed is load 2, the state in which the third softest load elastic body 31 is inserted and fixed is load 3, and the state in which the fourth softest (hardest) load elastic body 31 is inserted and fixed is load 4, as in the first use example in Example 2.

Furthermore, with regard to a procedure corresponding to the procedure C5 in the third use example in Example 1, for the third use example in Example 2, the three-axis acceleration sensor 11 and the pressure sensor 32 are activated, instructions are given to the subject such that the index finger is extended for three seconds from a state of 90-degree bent at the second joint, held in the maximum movable range for three seconds, and then returned to the original position for three seconds. The movement of the fingertip is measured while the instructions are repeated five times, the three-axis acceleration sensor 11 and the pressure sensor 32 are stopped after the measurement, and this procedure is thus almost the same as the procedure C5 in Example 1.

### [Example 3]

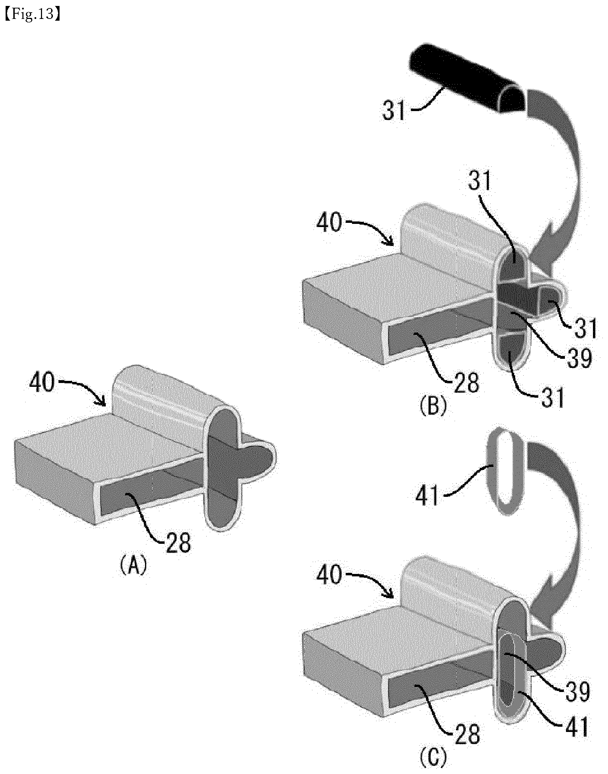

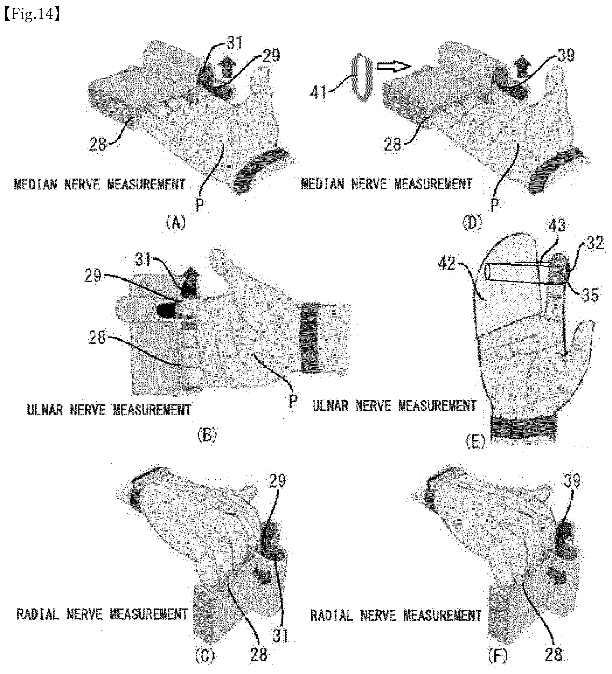

FIG. 13 is perspective views illustrating a structure of the manual muscle strength testing device according to Example 3, and FIG. 14 is diagrams illustrating the first to sixth use examples of the manual muscle strength testing device according to Example 3.

As illustrated in FIGS. 13 and 14, the manual muscle strength testing device according to Example 3 includes the following configurations (O) to (S). Some configurations are common to configurations in Example 1 or 2. The same numbers may be used and the explanation may be simplified for the common configurations. The three-axis acceleration sensor 11, the pressure sensor 32, and the ring 35 configured to fix these sensors illustrated in FIG. 11 are the same as the components used in Example 2, and the explanation is thus omitted (see (L) to (N) in Example 2).
(O) A finger insertion portion 40 that includes an other-finger fixation portion 28 having a space shaped such that the middle, ring, and little fingers of a subject can be inserted and the inserted fingers cannot move in any direction, and an index finger insertion portion 39 having a space shaped such that the index finger can be inserted and the inserted index finger can move to the ventral, thumb, or nail side.

The other-finger fixation portion 28 has the same configuration as in Example 2, and the index finger insertion portion 39 has a T-shaped tubular cross section into which the index finger can be inserted from both sides, as illustrated in FIG. 13(A).

Since the size of the fingers varies for individuals, a plurality of the finger insertion portions 40 with different lengths and heights of the other-finger fixation portion 28 are prepared in advance as with the finger insertion portion 30 in Example 2.
(P) A load elastic body 31 that can be inserted into, fixed to and removed from the protruding portion of the index finger insertion portion 39, except for a portion adjacent to the other-finger fixation portion 28. For details, see (K) in Example 2. FIG. 13(B) is a diagram illustrating a state in which the load elastic bodies 31 are inserted into and fixed to all the protruding portions.
(Q) A circular load elastic body 41 that can be fixed at one end to an end portion of the protruding portion of the index finger insertion portion 39 except for the portion adjacent to the other-finger fixation portion 28, and can be fixed by hooking the other end on the pressure sensor 32.

FIG. 13(C) is a diagram illustrating the overall shape and fixed state of the circular load elastic body 41.
(R) A bag-shaped other-finger fixation portion 42 (see FIG. 14(E)) having a space shaped such that the middle, ring, and little fingers of the subject can be inserted and the inserted fingers cannot move in any direction.
(S) A strap-type load elastic body 43 (see FIG. 14(E)) that can be fixed at one end to the outside of the little finger insertion portion of the bag-shaped other-finger fixation portion 42 and hooked on and fixed to the pressure sensor 32 at the other end.

FIG. 14(A) is a diagram illustrating the first use example of the manual muscle strength testing device according to Example 3.

In the first use example in Example 3, after the load elastic body 31 with appropriate softness is inserted into and fixed to the protruding portion of the index finger insertion portion 39, the palm P of the subject is turned upward, the middle, ring, and little fingers are inserted into the other-finger fixation portion 28 and the index finger is inserted into the index finger insertion portion 39.

The way of subsequent movement of the index finger, determination of the state of the flexor digitorum superficialis, evaluation of the state and the recovery level of the median nerve by analyzing the measured values, and the correspondence and comparison with the procedures A1 to A7 in the first use example in Example 1 are exactly the same as in the first use example in Example 2.

FIG. 14(B) is a diagram illustrating the second use example of the manual muscle strength testing device according to Example 3.

In the second use example in Example 3, after the load elastic body 31 with appropriate softness is inserted into and fixed to the protruding portion of the index finger insertion portion 39, the palm P of the subject is turned sideways, the middle, ring, and little fingers are inserted into the other-finger fixation portion 28 and the index finger is inserted into the index finger insertion portion 39.

The way of subsequent movement of the index finger, determination of the state of the dorsal interossei, evaluation of the state and the recovery level of the ulnar nerve by analyzing the measured values, and the correspondence and comparison with the procedures B1 to B5 in the second use example in Example 1 are exactly the same as in the second use example in Example 2.

FIG. 14(C) is a diagram illustrating the third use example of the manual muscle strength testing device according to Example 3.

In the third use example in Example 3, after the load elastic body 31 with appropriate softness is inserted into and fixed to the protruding portion of the index finger insertion portion 39, the palm P of the subject is turned downward, the middle, ring, and little fingers are inserted into the other-finger fixation portion 28 and the index finger is inserted into the index finger insertion portion 39.

The way of subsequent movement of the index finger, determination of the state of the extensor digitorum, evaluation of the state and the recovery level of the radial nerve by analyzing the measured values, and the correspondence and comparison with the procedures C1 to C5 in the third use example in Example 1 are exactly the same as in the third use example in Example 2.

FIG. 14(D) is a diagram illustrating a fourth use example of the manual muscle strength testing device according to Example 3.

The fourth use example in Example 3 is in common with the first use example of the manual muscle strength testing device according to Examples 2 and 3 in that the state and the recovery level of the median nerve can be evaluated by determining the state of the flexor digitorum superficialis and analyzing the measurement values. In the first use examples in Examples 2 and 3, after the load elastic body 31 with appropriate softness is inserted and fixed to the protruding portion of the index finger insertion portion 29 or 39, the fingers other than the thumb of the subject are inserted into the finger insertion portion 30 or 40, and then the index finger is moved while contracting the load elastic body 31 to measure the movement of the fingertip, whereas in the fourth use example in Example 3, the circular load elastic body 41 is fixed to the end of the protruding portion of the index finger insertion portion 39 at one end and is hooked on the pressure sensor 32 at the other end, the fingers other than the thumb of the subject are inserted into the finger insertion portion 40, and then the index finger is moved while extending the circular load elastic body 41 to measure the movement of the fingertip.

The way of movement of the index finger after the fingers other than the thumb are inserted into the finger insertion portion 40, determination of the state of the flexor digitorum superficialis, evaluation of the state and the recovery level of the median nerve by analyzing the measured values, and the correspondence and comparison with the procedures A1 to A7 in the first use example in Example 1 are almost the same as in the first use example in Example 2. With regard to a procedure corresponding to the procedure A5, however, for the fourth use example in Example 3, a state in which the other end of the circular load elastic body 41 is not hooked on the pressure sensor 32 is load 0, a state in which one end of the softest circular load elastic body 41 is fixed and the other end is hooked on the pressure sensor 32 is load 1, a state in which one end of the second softest circular load elastic body 41 is fixed and the other end is hooked on the pressure sensor 32 is load 2, a state in which one end of the third softest circular load elastic body 41 is fixed and the other end is hooked on the pressure sensor 32 is load 3, and a state in which one end of the fourth softest (hardest) circular load elastic body 41 is fixed and the other end is hooked on the pressure sensor 32 is load 4.

FIG. 14(E) is a diagram illustrating a fifth use example of the manual muscle strength testing device according to Example 3.

The fifth use example in Example 3 is in common with the second use example of the manual muscle strength testing device according to Examples 2 and 3 in that the state and the recovery level of the ulnar nerve can be evaluated by determining the state of the dorsal interossei and analyzing the measurement values. In the second use examples in Examples 2 and 3, after the load elastic body 31 with appropriate softness is inserted and fixed to the protruding portion of the index finger insertion portion 29 or 39, the fingers other than the thumb of the subject are inserted into the finger insertion portion 30 or 40, and then the index finger is moved while contracting the load elastic body 31 to measure the movement of the fingertip, whereas in the fifth use example in Example 3, the middle, ring, and little fingers are inserted into the bag-shaped other-finger fixation portion 42 to which one end of the strap-type load elastic body 43 is fixed, the other end of the strap-type load elastic body 43 is hooked on the pressure sensor 32, and then the index finger is moved while extending the strap-type load elastic body 43 to measure the movement of the fingertip.

The way of movement of the index finger, determination of the state of the dorsal interossei, evaluation of the state and the recovery level of the ulnar nerve by analyzing the measured values, and the correspondence and comparison with the procedures B1 to B5 in the second use example in Example 1 are almost the same as in the second use example in Example 2. With regard to a procedure corresponding to the procedure B4, however, for the fifth use example in Example 3, a state in which the other end of the strap-type load elastic body 43 is not hooked on the pressure sensor 32 is load 0, a state in which the other end of the softest strap-type load elastic body 43 is hooked on the pressure sensor 32 is load 1, a state in which the other end of the second softest strap-type load elastic body 43 is hooked on the pressure sensor 32 is load 2, a state in which the other end of the third softest strap-type load elastic body 43 is hooked on the pressure sensor 32 is load 3, and a state in which the other end of the fourth softest (hardest) strap-type load elastic body 43 is hooked on the pressure sensor 32 is load 4.

FIG. 14(F) is a diagram illustrating a sixth use example of the manual muscle strength testing device according to Example 3.

The sixth use example in Example 3 is in common with the third use example of the manual muscle strength testing device according to Examples 2 and 3 in that the state and the recovery level of the radial nerve can be evaluated by determining the state of the extensor digitorum and analyzing the measurement values. In the third use examples in Examples 2 and 3, after the load elastic body 31 with appropriate softness is inserted and fixed to the protruding portion of the index finger insertion portion 29 or 39, the fingers other than the thumb of the subject are inserted into the finger insertion portion 30 or 40, and then the index finger is moved while contracting the load elastic body 31 to measure the movement of the fingertip, whereas in the sixth use example in Example 3, the circular load elastic body 41 is fixed to the end of the protruding portion of the index finger insertion portion 39 at one end and is hooked on the pressure sensor 32 at the other end, the fingers other than the thumb of the subject are inserted into the finger insertion portion 40, and then the index finger is moved while extending the circular load elastic body 41 to measure the movement of the fingertip.

The way of movement of the index finger after the fingers other than the thumb are inserted into the finger insertion portion 40, determination of the state of the extensor digitorum, evaluation of the state and the recovery level of the radial nerve by analyzing the measured values, and the correspondence and comparison with the procedures C1 to C5 in the third use example in Example 1 are almost the same as in the third use example in Example 2. With regard to a procedure corresponding to the procedure C4, however, for the sixth use example in Example 3, the state in which the other end of the circular load elastic body 41 is not hooked on the pressure sensor 32 is load 0, the state in which one end of the softest circular load elastic body 41 is fixed and the other end is hooked on the pressure sensor 32 is load 1, the state in which one end of the second softest circular load elastic body 41 is fixed and the other end is hooked on the pressure sensor 32 is load 2, the state in which one end of the third softest circular load elastic body 41 is fixed and the other end is hooked on the pressure sensor 32 is load 3, and the state in which one end of the fourth softest (hardest) circular load elastic body 41 is fixed and the other end is hooked on the pressure sensor 32 is load 4.

### (Modification Examples of Examples 1 to 3)

Modification examples of the manual muscle strength testing device according to Examples 1 to 3 will be listed below.
(1) The side wall 5 in Example 1 has one opening 6 in the center. Alternatively, as with the palm rest 1, the side wall 5 may have a plurality of holes through which rubber bands can be passed, so that an end portion of the rubber band 14 can be hooked on the head of the pin 4 protruding from the bottom side of the plate constituting the palm rest 1.
(2) The restrainer 8 in Example 1 has the buckle 10 at one end of the flat belt 9. Alternatively, the restrainer 8 may be an elastic belt that can be removably attached to the top face of the palm rest 1, or a palm press with various sizes.
(3) In Example 1, the three-axis acceleration sensor 11 is used. Alternatively, when a displacement amount in one axial direction with respect to the movement of the fingertip (for example, a displacement amount in the Y-axis direction as in FIG. 6) is measured, a one-axis acceleration sensor or a two-axis acceleration sensor may be used, when a displacement amount in two axial directions with respect to the movement of the fingertip is measured, the two-axis acceleration sensor may be used.

Furthermore, a tilt sensor or a gyro sensor may also be used. These are collectively referred to as "inertial sensor" in the claims.
(4) The load applying portion 12 in Example 1 includes the binding bands 13, two rubber bands 14, and pins 4 fixed in two rows × seven holes 2. Alternatively, the load applying portion 12 may include an adjustable load applying mechanism, including an elastic member, a solenoid, or the like, that is placed on the palm rest 1 or the side wall 5 and capable of applying an adjustable load in the direction of the palm rest 1 or the side wall 5 to any one of the fingertip to be measured for movement, the three-axis acceleration sensor 11, and the ring 15.

Furthermore, the load applying portion 12 may be an adjustable load applying mechanism capable of removably attaching weights having various weights to the three-axis acceleration sensor 11 or the ring 15.

When such an adjustable load applying mechanism is used as the load applying portion, the holes 2 provided in the palm rest 1 or the opening 6 provided in the side wall 5 need not be prepared.
(5) In Example 1, the three-axis acceleration sensor 11 is fixed to the sensor fixation portion 18 of the ring 15 with a double-sided tape or an adhesive. Alternatively, the sensor fixation portion 18 may have a structure where the three-axis acceleration sensor 11 is removably attached to the sensor fixation portion 18, which is easier to use.
(6) In the first to third use examples in Examples 1 to 3 and the fourth to sixth use examples in Example 3, the state and the recovery level of the median, ulnar, and radial nerves are evaluated by determining the state of the flexor digitorum superficialis, dorsal interossei, and extensor digitorum, respectively. Alternatively, the state of the median nerve may be evaluated by determining the state of opponens pollicis (a muscle that presses the thumb and index finger to each other) or abductor pollicis brevis (a muscle that extends the thumb), the state of the ulnar nerve may be evaluated by determining the state of opponens digiti minimi (a muscle that presses the thumb and little finger to each other) or flexor digitorum profundus (a muscle that bends the little and ring fingers), and the state of the radial nerve may be evaluated by determining the state of wrist extensors (muscles that lift the entire back of the hand).
(7) In Example 2, the positions for fixing the three-axis acceleration sensor 11 and the pressure sensor 32 in the first and third use examples are different from the positions for fixing the three-axis acceleration sensor 11 and the pressure sensor 32 in the second use example. Alternatively, an acceleration sensor fixation portion is provided at a position of the ring 35 opposite to the side face sensor fixation portion 38, and then, in any of the use examples, the three-axis acceleration sensor 11 is fixed to the acceleration sensor fixation portion, the pressure sensors 32 are fixed to all of the upper face sensor fixation portion 36, the lower face sensor fixation portion 37, and the side face sensor fixation portion 38, thereby performing the measurements in the first to third use examples while the ring 35 is first worn on the fingertip.

Furthermore, even in Example 3, once the ring 35 is worn on the fingertip, all the measurements in the first to sixth use examples can be performed. In particular, in the first to third use examples, as illustrated in FIGS. 14(A) to 14(C), the relationship between the palm P of the subject and the finger insertion portion 40 is the same, only the direction of the palm P and the direction in which the index finger is moved differ, and the tests can be performed continuously in the first to third use examples after the fingers other than the thumb of the subject are inserted into the finger insertion portion 40, thereby significantly shortening the testing time.
(8) In Examples 2 and 3, the pressure sensor 32 is fixed to the ring 35 and the magnitude of the load applied to the fingertip is measured. Alternatively, the pressure sensor 32 need not be provided if the magnitude of each of the loads received by the fingertip from the load elastic body 31, the circular load elastic body 41, and the strap-type load elastic body 43 is measured in advance to grasp the magnitude of the loads for each softness of the elastic bodies.

### Reference Signs List

- 1: Palm rest
- 2: Hole
- 3: Slit
- 4: Pin
- 5: Side wall
- 6: Opening
- 7: Leg
- 8: Restrainer
- 9: Flat belt
- 10: Buckle
- 11: Three-axis acceleration sensor
- 12: Load applying portion
- 13: Rubber band placement portion
- 14: Rubber band
- 15: Ring
- 16: Circular portion
- 17: Cut
- 18: Sensor fixation portion
- 19: Through hole
- 20: Finger press
- 21: U-shaped portion
- 22: Finger press flat portion
- 23: Other-finger press
- 24: Semicircular portion
- 25: Other-finger press flat portion
- 26: Fingerstall
- 27: Protrusion
- 28: Other-finger fixation portion
- 29: Index finger insertion portion
- 30: Finger insertion portion
- 31: Load elastic body
- 32: Pressure sensor
- 33: Data transmission instrument
- 34: Signal transmission line
- 35: Ring
- 36: Upper face sensor fixation portion
- 37: Lower face sensor fixation portion
- 38: Side face sensor fixation portion
- 39: Index finger insertion portion
- 40: Finger insertion portion
- 41: Circular load elastic body
- 42: Bag-shaped other-finger fixation portion
- 43: Strap-type load elastic body
- H: Hand of examiner
- P: Palm
- δ: Amplitude
- δa: Average value of amplitude δ

## Claims

1. A manual muscle strength testing device comprising:
an other-finger fixation portion capable of fixing a middle finger, a ring finger, and a little finger of a subject;
an inertial sensor wearable on a fingertip of an index finger of the subject, being capable of measuring movement of the fingertip and recording or transmitting movement measurement data; and
a load applying portion that applies a load to the fingertip wearing the inertial sensor.

2. The manual muscle strength testing device according to claim 1, wherein the load applying portion is either an elastic body that is fixed to a ventral side of the fingertip and contracts with a force in a direction the fingertip moves to the ventral side, an elastic body that is fixed to a nail side of the fingertip and extends with a force in a direction the fingertip moves away from the nail side, an elastic body that is fixed to a thumb side of the fingertip and contracts with a force in a direction the fingertip moves to the thumb side, an elastic body that is fixed to an other-finger fixation portion side of the fingertip and extends with a force in a direction the fingertip moves away from the other-finger fixation portion, an elastic body that is fixed to the nail side of the fingertip and contracts with a force in a direction the fingertip moves to the nail side, or an elastic body that is fixed to the ventral side of the fingertip and extends with a force in a direction the fingertip moves away from the ventral side.

3. The manual muscle strength testing device according to claim 1 or 2, comprising a pressure sensor wearable on the fingertip, being capable of measuring magnitude of a load applied to either the ventral side, the thumb side, or the nail side of the fingertip and recording or transmitting load measurement data, wherein
the inertial sensor and the pressure sensor are fixed to a ring wearable on the fingertip,
the pressure sensor is fixed to all of or any one of the ventral side, the thumb side, and the nail side of the fingertip at an outside circular portion of the ring, and
the load applying portion applies a load to the pressure sensor fixed to either the ventral side, the thumb side, or the nail side of the fingertip.

## Amended claims

### Amended claims under Art. 19.1 PCT

1. (Amended) A manual muscle strength testing device comprising:
an other-finger fixation portion capable of fixing a middle finger, a ring finger, and a little finger of a subject;
an inertial sensor wearable on a fingertip of an index finger of the subject, being capable of measuring movement of the fingertip and recording or transmitting movement measurement data; and
a load applying portion that applies a load to the fingertip wearing the inertial sensor, wherein
the load applying portion is either an elastic body that is fixed to a ventral side of the fingertip and contracts with a force in a direction the fingertip moves to the ventral side, an elastic body that is fixed to a nail side of the fingertip and extends with a force in a direction the fingertip moves away from the nail side, an elastic body that is fixed to a thumb side of the fingertip and contracts with a force in a direction the fingertip moves to the thumb side, an elastic body that is fixed to an other-finger fixation portion side of the fingertip and extends with a force in a direction the fingertip moves away from the other-finger fixation portion, an elastic body that is fixed to the nail side of the fingertip and contracts with a force in a direction the fingertip moves to the nail side, or an elastic body that is fixed to the ventral side of the fingertip and extends with a force in a direction the fingertip moves away from the ventral side.

2. (Amended) The manual muscle strength testing device according to claim 1, comprising a pressure sensor wearable on the fingertip, being capable of measuring magnitude of a load applied to either the ventral side, the thumb side, or the nail side of the fingertip and recording or transmitting load measurement data, wherein
the inertial sensor and the pressure sensor are fixed to a ring wearable on the fingertip,
the pressure sensor is fixed to all of or any one of the ventral side, the thumb side, and the nail side of the fingertip at an outside circular portion of the ring, and
the load applying portion applies a load to the pressure sensor fixed to either the ventral side, the thumb side, or the nail side of the fingertip.

Statement under Art. 19.1 PCT
Claim 1 is amended by making originally filed claim 2 into an independent claim.

Claim 2 is amended by making originally filed claim 3 into a dependent claim depending only from claim 1.
